(19) Europäisches Patentamt
European Patent Office
Office européen des brevets

(11) Publication number : **0 467 701 A2**

# (12) EUROPEAN PATENT APPLICATION

(21) Application number : **91306599.1**

(22) Date of filing : **19.07.91**

(51) Int. Cl.$^5$ : **C07K 7/08,** C07K 7/10, C12P 21/04, A61K 37/02, A61K 39/21

(30) Priority : **19.07.90 US 555558**
**19.06.91 US 715127**

(43) Date of publication of application :
**22.01.92 Bulletin 92/04**

(84) Designated Contracting States :
**AT BE CH DE DK ES FR GB IT LI LU NL SE**

(71) Applicant : **MERCK & CO. INC.**
**126, East Lincoln Avenue P.O. Box 2000**
**Rahway New Jersey 07065-0900 (US)**

(72) Inventor : **Bondy, Steven S.**
**12 High Court**
**High Bridge, NJ 08829 (US)**
Inventor : **Emini, Emilio A.**
**6 Faggs Manor Lane**
**Paoli, PA 19301 (US)**
Inventor : **Marburg, Stephen**
**50 Concord Avenue**
**Metuchen, NJ 08840 (US)**
Inventor : **Tolman, Richard L.**
**29 Upper Warren Way**
**Warren, NJ 07059 (US)**
Inventor : **Hannah, John**
**155 Idlebrook Lane**
**Matawan, NJ 07747 (US)**

(74) Representative : **Barrett-Major, Julie Diane et al**
**Merck & Co., Inc. European Patent Department Terlings Park Eastwick Road**
**Harlow Essex CM20 2QR (GB)**

## (54) Cyclic HIV principal neutralizing determinant peptides.

(57) Human Immunodeficiency Virus (HIV) Principal Neutralizing Determinant (PND) peptides, or peptides immunologically equivalent therewith, having the structure (SEQ ID : 1 :) :

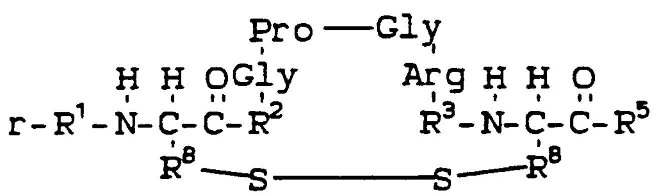

wherein the ring system contains disulfide bonds, are useful as immunogens, as analytical tools, as reagents in ELISA assays, or as reagents for making covalent conjugate immunogens. Conjugates containing these cyclic peptides are useful for raising mammalian anti-peptide, anti-HIV, or HIV-neutralizing immune responses, and a composition containing such a conjugate may be used as a vaccine to prevent HIV disease, including Acquired Immune Deficiency Syndrome, (AIDS), or AIDS related complex, (ARC), or as an immunogen for treating humans afflicted with AIDS or ARC.

EP 0 467 701 A2

## BACKGROUND OF THE INVENTION

Human Immunodeficiency Virus (HIV) principal neutralizing determinant (PND) peptides have been described which are capable of raising an anti-HIV immune response in mammals. The hypervariable region of the AIDS virus envelope glycoprotein, gp120, between amino acids 296 and 341 [according to the numbering scheme of Ratner et al., Nature 313, 277 (1985)] contains the amino acid tetramer -Gly Pro Gly Arg- (SEQ ID: 2:) in most of the HIV isolates identified to date. In addition, a cysteine residue is generally found within about 20 amino acids on either side of this tetramer. In at least one common isolate, HIV IIIB, it is known that these cysteines are disulfide bonded. Thus, the intermediate amino acids, herein referred to as loop amino acids, are forced into a cyclic structure, with the -GPGR- being exposed at the loop-tip [Javaherian et al., PNAS USA 86, 6768, (1989)].

Peptide based efforts aimed at the induction of HIV neutralizing immune responses in mammals have generally been limited to the use of linear or disulfide bonded cyclic peptides. The linear peptides have the limitation that the recipient immune system is exposed to an epitope which has a large number of solution 3-dimensional conformations which are local energy minima. Although reliance on disulfide bonding to limit the number of conformations assumable by the linear PNDs is not completely satisfactory as disulfides may be labile under certain conditions, and breakage of the disulfides results in the linear peptide having the attendant problems described above, these peptides have the virtue of being easily prepared by oxidation of sulfhydryl containing peptides. Furthermore, preferred embodiments of the instant invention are found to be surprisingly active in the induction of HIV neutralizing antibodies.

Linear synthetic peptides have been prepared by a number of strategies conducted either in solution or on solid supports. Excellent texts covering the principles and techniques involved are listed: Principles of Peptide Synthesis, Bodanszky. M., Springer-Verlag (1984); Solid Phase Peptide Synthesis, Stewart J. M., Young, J. D., Pierce Chemical Company (2nd. ed. 1984); The Peptides, Gross, E., Meienhofer, J., Academic Press, Inc., (1979). In general, where cyclic peptides are required, disulfide-bonded cyclics have been prepared by oxidation of linear synthetic peptides containing at least two sulfhydryls, for example, peptides containing two cysteines. The instant invention provides new disulfide-bonded cyclic HIV peptides.

Thus, this invention discloses novel HIV PND peptides and their derivatives which comprise disulfide-bonded cyclic structures, a process for making, and a method of using such compounds. The cyclic HIV PND peptides, cPNDs, are prepared by formation of a disulfide through cysteines on ether side of the loop amino acids. The cPNDs so formed are useful as free peptidyl immunogens or as reagents for preparing covalent conjugate immunogens containing the cPNDs. Such conjugates are useful for raising mammalian anti-peptide, anti-HIV, or HIV-neutralizing immune responses. Compositions containing such conjugates may be used as a vaccine to prevent HIV disease, including Acquired Immune Deficiency Syndrome, (AIDS), or AIDS related complex (ARC) or as an immunogen for treating humans afflicted with AIDS or ARC. In addition, such conjugates provide a useful laboratory tool for analyzing the structure-function relationship involved in peptide elicitation of mammalian HIV-neutralizing immune responses.

## SUMMARY OF THE INVENTION

This invention is concerned with novel, cyclic HIV PND peptides having the structure (SEQ ID: 1:):

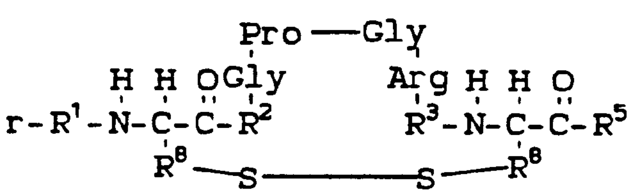

or pharmaceutically acceptable salts thereof, wherein:

r is:

a) hydrogen,

b)

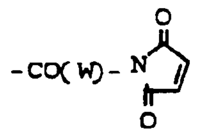 ,

wherein W is preferably $-(CH_2)_2-$ or $-(CH_2)_3-$ or $R^6$, where $R^6$ is

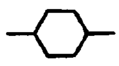 , 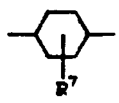 , 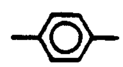 or 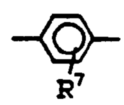

wherein $R^7$ is lower alkyl, lower alkoxy, or halo;

$R^1$ is:

a) a bond, or

b) a peptide of 1 to 5 amino acids, optionally including a marker amino acid, preferably Nle, Abu.

$R^2$ is:

a peptide of 3 to 10 amino acids

$R^3$ is:

a peptide of 3 to 10 amino acids

$R^5$ is:

a) -OH,

b) a peptide of 1 to 5 amino acids, optionally including a marker amino acid,

c) $-NH_2$;

$R^8$ is lower alkyl of between one and eight carbons.

Peptides of this invention are prepared by cyclizing linear peptides made by solid phase chemical synthesis incorporating protective group chemistry. Cyclization is achieved by inclusion, during synthesis of the linear peptide, of reactive sites having labile protecting groups on the amino-and carboxy-terminal sides of the loop amino acids. Thus, in one embodiment of the invention, the sulfurs of cysteines incorporated into the peptide on either side of the loop amino acids are oxidized to the disulfide. The cyclic peptides may then be used as analytical tools, as reagents in ELISA assays, or as reagents for conjugation to an immunogenic carrier which may be comprised of polysaccharide, protein, both polysaccharide and protein, or any other material which confers enhanced immunogenicity on the cyclic peptide. The peptides may also be used directly, in an unconjugated state, as immunogens for the elicitation of anti-peptide, anti-HIV, or HIV-neutralizing immune responses in a mammal.

In the preparation of conjugates using the peptide of this invention, any of a number of means known in the art may be used to link the carrier protein with the peptide. In a preferred embodiment of the invention, a N-maleimido alkanoic acid N-hydroxysuccinimido ester (NHS ester) or N-maleimido-$R^6$- carboxylic acid N-hydroxysuccinimido ester, wherein the N-maleimido propionic acid NHS ester is preferred, is reacted with a free peptide amino group in an aqueous buffer, preferably aqueous bicarbonate at about pH 8, or in DMF containing about an equivalent of diethyl isopropyl amine, to generate the maleimidated peptide. The maleimidated peptide is then amenable to reaction with a thiolated carrier, such as a protein, or polysaccharide, preferably the OMPC of Neisseria meningitidis B, the major immune enhancing protein (MIEP), or to polyribosyl ribitol phosphate which in turn is linked to a carrier protein.

Such conjugates are useful for inducing mammalian anti-peptide, anti-HIV, or HIV-neutralizing immune responses and for formulating vaccines to prevent HIV-Disease, including AIDS or ARC or for treating humans afflicted with AIDS or ARC.

## OBJECTS OF THE INVENTION

Accordingly, it is an object of this invention to provide novel, and cyclic HIV PND peptides which are useful as free immunogens or as reagents for preparing conjugate immunogens which may be used to elicit mammlian anti-peptide, anti-HIV, or HIV-neutralizing immune responses, or to prepare compositions for use as anti-HIV vaccines or as immunogens for treatment of humans afflicted with AIDS. Another object is to provide a process for the cyclization of HIV PND peptides through disulfide bonded structures.

DEFINITIONS AND ABBREVIATIONS

| | |
|---|---|
| AA assay | amino acid analysis method wherein peptides or proteins are acid hydrolyzed to the free amino acids and then quantitated |
| Acm | acetamidomethyl thiol protecting group |
| activation | reaction of peptides, proteins, or polysaccharide moieties with a reagent capable of derivatizing the moiety in order to enable subsequent desirable reactions to occur |
| AIDS | Acquired Immune Deficiency Syndrome |
| amino acid | a molecule having both an acid and amino functional group; there are 20 common α-amino acids with the general structure $H_2N$-CHR-COOH, wherein the R group defines the identity of the amino acid; these amino acids may have either a D or L stereochemical form and unless specified otherwise, by the lower-case one letter abbreviation, or the prefix D-before the amino acid name, the amino acid is of the natural or L configuration; the names of the 20 common amino acids and the structure of the R group are identified herein in single-letter code according to the following table: |

| AMINO ACID NAME | 3-letter code | 1-letter code | side-chain (R) |
|---|---|---|---|
| Alanine | Ala | A | $-CH_3$ |
| Arginine | Arg | R | $-(CH_2)_3NHCHNH_2NH_2^+$ |
| Asparagine | Asn | N | $-CH_2CONH_2$ |
| Aspartic Acid | Asp | D | $-CH_2COOH$ |
| Cysteine | Cys | C | $-CH_2SH$ |
| Glutamic Acid | Glu | E | $-(CH_2)_2COOH$ |
| Glutamine | Gln | Q | $-(CH_2)_2CONH_2$ |
| Glycine | Gly | G | -H |
| Histidine | His | H | $-CH_2$-imidazole |
| Isoleucine | Ile | I | $-CH(CH_3)CH_2CH_3$ |
| Leucine | Leu | L | $-CH_2CH(CH_3)_2$ |
| Lysine | Lys | K | $-(CH_2)_4NH_3^+$ |
| Methionine | Met | M | $-(CH_2)_2SCH_3$ |
| Phenylalanine | Phe | F | $-CH_2$-Phenyl |
| Proline | Pro | P | -α, N-$(CH_2)_3$ |
| Serine | Ser | S | $-CH_2OH$ |
| Threonine | Thr | T | $-CH(OH)CH_3$ |
| Tryptophan | Trp | W | $-CH_2$-indole |
| Tyrosine | Tyr | Y | $-CH_2$-phenyl-OH |
| Valine | Val | V | $-CH(CH_3)_2$ |

| | |
|---|---|
| antibody | a protein produced by mamalian B cells that is capable of binding a particular antigen |
| ARC | AIDS-Related Complex |
| AZT | Azidothymidine, an anti-AIDS compound |
| bigeneric spacer | a molecular chain resulting from the reaction of separately derivatized partners; analytical degradation of the conjugate formed through the spacer allows release and quantitation |

| | |
|---|---|
| | of the spacer, providing a measure of the degree of covalent attachment |
| capping | the elimination of reactive sites on a conjugate by reaction with small molecules |
| Cbz | benzyloxycarbonyl |
| conjugate | a complex of discrete chemical entities covalently bound one to the other, wherein at least one entity is a desired antigen (e.g. an HIV PND) and another entity is a carrier |
| Core amino acids | those amino acids of an HIV PND which are essential for inducing HIV-neutralizing immune responses in a mammal |
| DPPA | diphenylphosphorylazide |
| ELISA | enzyme-linked immunosorbant assay |
| Fmoc | 9-fluorenylmethyloxycarbonyl |
| HIV | Human Immunodeficiency Virus, a member of the lentivirus group and the purported etiologic agent implicated in AIDS and related complexes; HIV is alternatively known as HTLV (Human T-cell Lymphocyto-trophic Virus), LAV (Lymphadenopathy Associated Virus), and ARV (AIDS Related Virus) |
| immunogen | a molecule useful as a stimulator of a mammalian immune response |
| immunologically equivalent peptides | cyclic or linear peptides having in common the function of eliciting HIV neutralizing immune responses in mammals, such as antibodies which are able to recognize the equivalent peptide epitopes |
| marker amino acid | an amino acid having a signal in the AA assay which is free of interference by signals generated by other peptide or protein amino acids |
| Mtr | 4-methoxy-2,3,6-trimethylphenyl sulfonyl |
| NEM | N-ethylmaleimide |
| OMPC | Outer Membrane Protein Complex of <u>Neisseria</u> <u>meningitidis</u>; used as an immunoenhancer and peptide carrier |
| peptide | a polymer of amino acids linked by amide (peptide) bonds |
| PEP | peptide |
| PND | Principal Neutralizing Determinant; the name attributed to peptidyl sequences capable of high-affinity binding to HIV neutralizing antibodies and capable of raising HIV-neutralizing antibodies in a mammalian recipient upon inoculation with an immunogen containing the PND |
| PnPs6B | <u>Streptococcus</u> <u>pneumoniae</u> 6B capsular polysaccharide |
| PRO | an immunogenic protein |
| protein | a large peptide |
| PRP | Polyribosyl-ribitol phosphate |
| PSA | anionic polysaccharide, usually having repeat phosphate units in the monomer unit of the polymer |
| resins | solid support matrices for solid phase peptide synthesis<br><u>Wang</u>:<br>4-(hydroxymethyl)phenoxymethyl linkage to copolystyrene-1%divinylbenzene resin, which is used for batch Fmoc solid phase peptide synthesis, with final 95% TFA cleavage from the resin and concomitant deprotection of acid sensitive side chain protecting groups;<br><u>Sasrin</u>:<br>4-(hydroxymethyl)-3-methoxyphenoxymethyl linkage to copolystyrene-1%divinylbenzene resin, which is used for batch Fmoc solid phase peptide synthesis, with final 1% $TFA/CH_2Cl_2$ cleavage from the resin, leaving intact acid labile side chain protecting groups;<br><u>Pepsyn KH</u>:<br>4-(hydroxymethyl)phenoxymethyl linkage to polyamide resin adsorbed on to kieselguhr, which is used for continuous flow column Fmoc solid phase peptide synthesis. Peptides are cleaved from the resin as described above for Wang resin;<br><u>Pepsyn KB</u>:<br>4-(hydroxymethyl)-3-methoxymethyl linkage to polyamide resin adsorbed on to kieselguhr, which is used for Fmoc solid phase peptide synthesis. Side chain protected peptides are cleaved from the resin as described above for the Sasrin resin |
| "scaffold" | immunogen having multiple peptide epitopes built upon a carrier molecule |
| SCMHC | S-carboxymethyl homocysteamine, an acid-stable bigeneric spacer released by degra- |

| | |
|---|---|
| | dation of covalent conjugate immunogens and quantifiable by AA assay |
| SCMC | S-carboxymethyl cysteamine, an acid-stable bigeneric spacer released by degradation of covalent conjugate immunogens and quantifiable by AA assay |
| Z | benzyloxycarbonyl |

## DETAILED DESCRIPTION OF THE INVENTION

This invention is concerned with novel cyclic HIV PND peptides having the structure (SEQ ID: 1:):

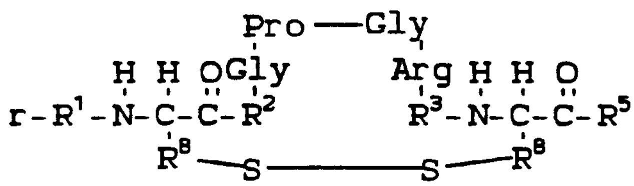

or pharmaceutically acceptable salts thereof, wherein:

r is:

a) hydrogen,

b)

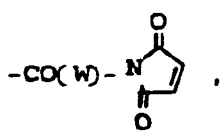 ,

wherein W is preferably $-(CH_2)_2-$ or $-(CH_2)_3-$ or $R^6$, where $R^6$ is

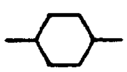 , 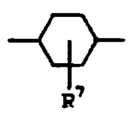 , 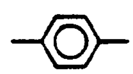 or 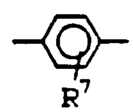

wherein $R^7$ is lower alkyl, lower alkoxy, or halo;

$R^1$ is:

a) a bond, or

b) a peptide of 1 to 5 amino acids, optionally including a marker amino acid;

$R^2$ is :

a peptide of 3 to 10 amino acids

$R^3$ is:

a peptide of 3 to 10 amino acids

$R^5$ is:

a) -OH,

b) a peptide of 1 to 5 amino acids, optionally including a marker amino acid, or

c) $-NH_2$;

$R^8$ is lower alkyl of between one and eight carbons.

Lower alkyl consists of straight or branched chain alkyls having from one to eight carbons unless otherwise specified. Hereinafter, amino acids $-R^2$-Gly Pro Gly Arg-$R^3$- (SEQ ID: 1:), which go toward formation of the loop of a cyclic peptide, will be referred to as loop amino acids.

In one embodiment of the invention, the cyclic peptide having the structure (SEQ ID:3:):

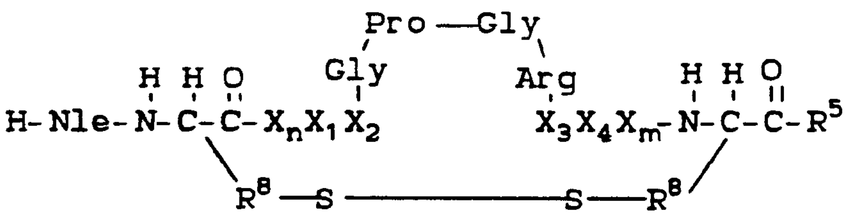

is prepared by cyclizing a linear peptide having the structure (SEQ ID: 3:):

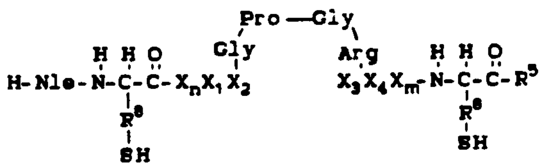

wherein:
$X_1$ is a constituent of $R^2$ selected from:
a) serine,
b) proline,
c) arginine,
d) histidine,
e) glutamine, which is preferred, or
f) threonine;
$X_2$ is a constituent of $R^2$ selected from:
a) isoleucine, which is most preferred,
b) arginine, which is preferred,
c) valine, or
d) methionine;
$X_n$ is a constituent of $R^2$ and is an amino acid or a peptide of up to 8 amino acids;
$X_3$ is a constituent of $R^3$ selected from:
a) alanine,
b) arginine, or
c) valine;
$X_4$ is a constituent of $R^3$ and is selected from:
a) phenylalanine,
b) isoleucine,
c) valine, or
d) leucine;
$X_m$ is a constituent of $R^3$ and is an amino acid or a peptide of up to 8 amino acids.
$X_2$ is preferably Isoleucine.

The novel cyclic peptides of this invention are prepared in essentially two phases: First the linear peptide is synthesized on a Milligen 9050 or an ABI-431A peptide synthesizer using 9-fluorenyl-methyloxycarbonyl (Fmoc) chemistry and appropriately side-chain protected Fmoc-amino acid pentafluorophenyl esters as reagents or using derivatized Wang resin, Fmoc chemistry, and side-chain protected Fmoc-amino acid symmetrical anhydrides, prepared in situ, as reagents.

Second, the linear peptide is cyclized, either in solution or with the peptide still attached to the solid phase resin by incorporating cysteine residues into the linear peptide at either end of the sequence which is to form the loop, and oxidizing these to the disulfide. In a preferred embodiment, cyclization is accomplished by exposure of the peptide to (a) $H_2O_2$, (b) atmospheric oxygen in an organic solvent such as methanol or $CH_3CN$, (c) aqueous $CH_3CN$ containing about 0.1 - 0.5% TFA, or (d) about 0.1M ferricyanide. The preferred method is exposure to atmospheric oxygen after removal of oxygen scavangers by column chromatography in an organic solvent or by other suitable means known in the art.

Products obtained may be characterized by fast atom bombardment-mass spectrometry [FAB-MS], reverse phase HPLC, amino acid analysis or nuclear magnetic resonance spectroscopy (NMR).

Thus, the peptides of this invention may be prepared as further described below in (i) and (ii):

i. Peptide Cyclization in the Solid State:

A linear peptide containing $C^1$ and $C^2$ on either side of the loop amino acids, where $C^1$ and $C^2$ are both cysteine or another amino acid containing free sulfhydryl groups in the side chain, is prepared according to known synthetic procedures (see discussion supra). In the completed cyclic PND, the sulfhydryl containing side chains, (-$R^8$-SH), go toward making up the -$R^8$-S-groups of the completed cyclic HIV PND structure shown above. Amino acids to be incorporated which have reactive side chains (R groups) are used in an appropriately R-group protected form. For example, histidine is triphenylmethyl (Trt), or Boc protected, and arginine is 4-methoxy-2,3,6-trimethylphenyl sulfonyl (Mtr) protected.

Preferably, a resin is purchased with $C^2$ in its Acm protected form already attached to the resin, for example, Fmoc-L-Cys(Acm)-O-Wang resin. The cysteine incorporated at the amino terminal side of the loop amino acids, $C^1$, may also be the Acm derivative. Either $C^1$ or $C^2$ may be bound to additional amino acids, $R^1$ or $R^5$ respectively, which may be utilized in the formation of conjugates with carrier molecules or may serve as marker amino acids for subsequent amino acid analysis, such as when norleucine or ornithine is used.

The sulfur of the acetamidomethylated cysteines are reacted, at room temperature for about 15 hours in a solvent compatible with the resin, as a 1-50% concentration of an organic acid, preferably about 10% acetic acid in anhydrous dimethylformamide (DMF), with about a four fold molar excess of a heavy metal salt, such as mercuric acetate [$Hg(OAc)_2$] for each Acm group. The resulting heavy metal thioether, for example the mercuric acetate thioether of the peptide, PEP(S-HgOAc), is then washed and dried. Addition of excess hydrogen sulfide in DMF yields insoluble metal sulfide, e.g. mercuric sulfide (HgS), and the peptide with free sulfhydryl groups. The free sulfhydryls are then oxidized by one of the aforementioned methods. Alternatively, the Acm protected thiols may be converted directly to the cyclic disulfide by treatment with iodine in a methanol/DMF solvent.

ii. Cyclization of Peptides in Solution:

Essentially the same process described above for solid state cyclization applies with two main variants: If the peptide is cleaved (95% TFA/4% ethanedithiol/1% thioanisole) from a pepsyn KA resin, acid labile side chain protecting groups are also removed, including Cys(Trt) which provides the necessary free -SH function. If however, Cys(Acm) protection is used, then mercuric acetate/hydrogen sulfide cleavage to the free -SH group is required as an independent procedure, with the linear peptide either on or off the resin.

One method however, is the use of Cys(Acm) protection and Sasrin or Pepsyn KH resin, and cleavage of the linear, fully protected peptide from the resin with 1% TFA/$CH_2Cl_2$. Mercuric acetate/hydrogen sulphide then selectively converts Cys(Acm) to the free -SH group, and cyclization is effected on the otherwise protected peptide. At this point, the peptide may be maleimidated in situ , selectively on the N-terminus. Acid labile side chain protecting groups are cleaved with 98% TFA/2% thioanisole, and the cyclic peptide is isolated by HPLC. The preferred method, however, is to cleave the peptide from the resin, and allow cyclization by one of the aforementioned methods. The most preferred method is to allow air oxidation for about one to fifty hours of between 10 and 40°C.

The following examples are provided to further define the cyclic peptide invention and to more particularly demonstrate how to make and use such peptides. However, the examples provided are not to be construed so as to limit the scope of the invention.

EXAMPLE 1

PREPARATION OF cPND33: (SEQ ID:4 :)

1. SYNTHSIS OF:

H-Nle Cys Tyr Asn Lys Arg Lys Arg Ile His Ile Gly Pro Gly Arg Ala Phe Tyr Thr Thr Lys Asn Ile Ile Gly Cys-OH ($C_{135}H_{220}N_{42}O_{33}S_2$, formula weight = 3023.6)

The 26mer was assembled on the Milligen # 9050 synthesizer, starting from partially racemised Fmoc-L-Cys(Trt)-OPKA resin (Milligen batch B 090426, 0.081 meq/g), using 2.47 g (0.200 meq). The theoretical yield is 604 mg. The resin was mixed with an equal volume of glass beads (Sigma 150-212 μm). The mixture completely filled two 1 x 10 cm columns, connected in series. Reagents were Fmoc-$Pf_P$ ester (except for threonine, which was dHBt), using four fold molar excess in N-methyl pyrrolidine solvent. Side chain protection was: Tyr (tert-butyl); Lys (Boc); Arg (Mtr); His (Boc); Thr (tert-butyl); Cys (Trt). The protocol was modified to give double coupling with $Lys^7$; $Ile^9$: $Ile^{11}$; $Gly^{12}$; $Pro^{13}$; $Gly^{14}$; $Arg^{15}$; $Phe^{17}$; $Tyr^{18}$; $Thr^{19}$; $Thr^{20}$; $Ile^{23}$; $Ile^{24}$. Acylation recycle

times were extended from 30 to 60 mminutes for all units, except for Gly[14] and Ala[16], and to 90 minutes for Ile[9] (2x); Ile[11] (2x); Ile[23] (2x) and Ile[24] (2x). The derivatized resin was maintained as the free terminal amine which was washed with $CH_2Cl_2$ and air-dried.

The mixture of dry derivatized resin and glass beads was resuspended in 95% TFA, 4% ethane dithiol, 1% $CH_3SPh$ (30 mL) at 23°C in a sealed flask, with gentle stirring on an oscillating tray for 8 hours. The bright yellow mixture was then filtered and the insolubles were thoroughly extracted with 100% TFA (3 x 20 mL). The combined dark orange filtrates were evaporated to give a pale tan, oily gum. On trituration with ether (20 mL) this material instantly became a colorless solid, which was transferred to a filter by triturating with additional ether (3 x 20 mL). After drying, the crude product was obtained as a fine colorless powder (583 mg).

Analytical reverse phase HPLC [aqueous 0.1% TFA/22% $CH_3CN$, $\lambda$ = 215 nm, A = 0.05, 2.0 mL/min.] on a 0.46 x 25.0 cm Vydac $C_{18}$ column of about a 50 µg sample, dissolved in 50 µL aqueous 0.1 % TFA/20% $CH_3CN$, 4 µL injected, revealed a major component (36.29′) and a later eluting minor component. These were separately collected after injection of a 30 mg and another 50 mg aliquot of the sample onto two 2.21 x 25.0 cm preparative Vydac $C_{18}$ columns in series [linear gradient over 60′: 0.1% TFA/23-27% $CH_3CN$, $\lambda$ = 215 nm, A = 3.00, 10 mL/min]. A total of 35.2 mg of the earlier eluting material (44.45′) and 8.2 mg of the later eluting material was recovered following lyophilization. FAB-MS of the major product gave a $[M+H]^+$ = 3022.1 and an $[M+Na]^+$ = 3044.2, which is consistent with the calculated mass.

2. PREPARATION OF THE CYCLIC DISULFIDE, cPND33: (SEQ ID:4:):

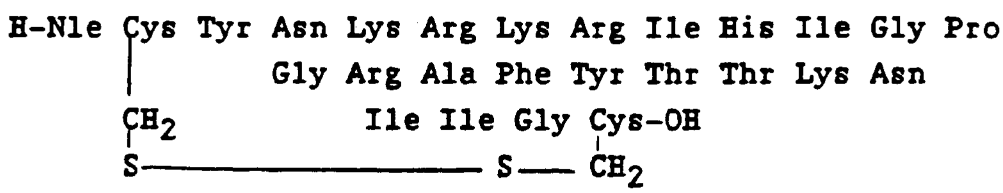

a. $K_3Fe(CN)_6$ INDUCED OXIDATION:

The linear 26 mer dithiol compound (35.0 mg) was dissolved in degassed distilled water (38 mL) at 23 °C to give a clear colorless solution at pH 2.73. The pH was adjusted to 8.5 with 0.1 N $NH_4OH$, and the solution was covered with an atmosphere of nitrogen. An aliquot of the material was immediately run on analytical reverse phase HPLC and found to be undergoing oxidation as evidenced by the appearance of a early peak.

With magnetic stirring, a freshly prepared solution of 0.01 M $K_3Fe(CN)_6$ was added by power driven hypodermic syringe at 23° C under nitrogen. Analysis of a small aliquot by HPLC revealed total conversion of starting material to an earlier elution time. The reaction mixture (pH 8.3) was mixed with 10% aqueous acetic acid and stirred to give a pH of 4.0. The solution was filtered to remove insoluble material, and the faintly yellow solution was evaporated and then lyophilized to give about 27.9 mg of a pale yellow powder. The material was dissolved in 0.1% TFA/20% $CH_3CN$ and gradient eluted on a preparative HPLC. A major early eluting peak and a later eluting peak (4:1) were separately collected and lyophilized to yield 6.1 mg of the early and 1.5 mg of the late eluting material. FAB-MS analysis of the early eluting material: $[M+H]^+$ 3019.7; $[M+Na]^+$ 3042.5; FAB-MS analysis of the late eluting material: $[M+H]^+$ 3020.0; $[M+Na]^+$ early material = 3041.5; all of which corresponds to the correct mass for the cyclized cPND33. The later eluting material is the D-cysteine carboxy terminus diastereomer.

Amino acid analysis of the products gave the predicted amino acid compositions for the cyclized products and confirmed that the later eluting material is the D-cysteine containing diastereomer.

b. AIR OXIDATION:

The linear 26 mer prepared in (1) above (86 mg, 28.4 µmoles) was dissolved in aqueous 0.1% TFA/20% acetonitrile (284 mL) at 23° C and the solution was allowed to stand open to the air. Cyclization was monitored by reverse phase HPLC and the sample was found to be almost completely converted to the early eluting material, with almost complete dissappearance of starting linear material, by t = 24 hours. The clear, colorless solution was evaporated to about 8 mL at which point an additional 10 mg sample prepared in the same way as the 86 mg, was added. The combined sample was evaporated to about 9 mL. The cloudy colorless solution was subjected to HPLC separation, in two separate runs, on two 2.12 x 25.0 cm Vydac $C_{18}$ columns in series. Two fractions were separately collected, an early eluting peak and a later eluting peak. Each peak was separately evaporated and lyophilized to yield 30.1 mg and 9.7 mg of the early and late materials respectively. The early eluting material was combined with other preparations of early eluting cyclized material to yield a total of 47.5 mg of a faintly bluish fluffy powder. Analytical HPLC of this material gave a single peak.

## EXAMPLE 2

### CONJUGATION OF OMPC WITH cPND33:

### 1. PREPARATION OF 3-MALEIMIDOPROPIONIC ACID ANHYDRIDE

3-Maleimidopropionic acid (226 mg) was covered with 5 mL of acetic anhydride and the mixture was heated at 130°C for 3.75 hr, and then aged over night at room temperatue. The solution was concentrated to an oil and the NMR spectrum ($CDCl_3$) indicated a mixture of the homoanhydride and the mixed anhydride of acetic and maleimidopropionic acids. The starting acid shows the methylene adjacent to the carbonyl as a triplet centered at 2.68 ppm whereas in the anhydride these resonances appear at 2.81 ppm. Purification was effected by fractional sublimation, first at 70°C and 0.2 mm and then at 120°C and 0.2 mm. The latter fraction was removed from the apparatus by dissolving in $CDCl_3$, affording 34 mg of pure homoanhydride on evaporation of the solvent. This was recrystallized from $CDCl_3$ and cyclohexane affording material melting at 143-147°C.
Calcd. for $C_{14}$ $H_{12}N_2O_7$: C,52,51;H,3.78;N,8.75.
Found: C,51.73;H3.67;N,8.16. 200 MHz NMR
($CDCl_3$):2.83 (2H,t)3.84 (2H,t),6.73 (2H,s).

### 2. "SELECTIVE" ACYLATION OF cPND33

cPND33 (22.5 mg; at estimated 70% peptide is equivalent to 15.75 mg or 5.212 micromoles) was dissolved in 12.0 mL of a 0.1M pH 5.25 morpholinoethane sulfonic acid buffer and cooled in an ice bath. Analysis of this solution and progress of the reaction was followed by HPLC on a 25 cm ODS column using 25% aqueous acetonitrile: 0.1% trifluoroacetic acid (TFA) as eluent.
Maleimidopropionic acid anhydride (2.0 mg, 6.25 micromoles) was dissolved in 0.600 mL of dry tetrahydrofuran, and 0.5 mL of this solution (corresponding to 5.2 micromoles of anhydride) was added to the above peptide solution. After 30 sec., a 7 microliter aliquot was removed and evaluated by HPLC. This assay was repeated at 0.25, 0.50, 1.25, 2.25 and 3.0 hr. After 3.5 hr the solution was lyophilized. The lyophilizate was dissolved in 2.0 mL of 20% aqueous acetonitrile, filtered through a 0.2 micron filter and preparatively chromatographed in three 0.700 mL runs on a 21.2 mm x 25 cm Zorbax C-18 column. The following elution program was used: flow rate = 10 mL/min; isocratic elution with 25% aqueous acetonitrile/0.1% TFA (12 min); gradient to 28% acetonitrile (10 min); gradient to 35% acetonitrile (8 min). The tail fractions were isolated by concentration and lyophilization to afford 8.9 mg of recovered starting material (penultimate fraction) and 9.6 mg of a product which had a mass spectrum (FAB) indicating a molecular weight of 3172 (i.e the mono-maleimidopropionyl derivative of cPND33).

The product was further characterized by a sequence analysis looking for the absence of lysine (the absence of any sequence would imply terminal amino acylation). The results indicate that most but not all of the maleimidopropionyl moiety is bonded to the lysine closest to the carboxy terminus.

### 3. CO-CONJUGATION OF MALEIMIDATED cPND33 WITH THIOLATED OMPC

#### A. Small Scale Experiment:

An aqueous 3-maleimidopropionic acid (MPA) solution (1 mg/mL) was prepared. This was titered as follows: to 2.98 mL of a solution of N-acetylcysteine (0.2 micromoles/mL in pH8.0 $PO_4$ buffer) was added 0.02 mL of the maleimidopropionic acid solution. After ageing for 10 min, 0.100 mL of Ellman reagent was added. The O.D. was determined at 412 nm using this material in the reference beam and a "blank" (prepared by substituting water for the sample) in the sample beam. A titer of 5.0 micromoles /mL of 3-maleimidopropionic acid was found by this Ellman "extinction" assay. The maleimidated peptide (MPP) from above (9.6 mg/0.800 mL of water) was titred in the same way and found to have 2.7 micromoles/mL.

OMPC thiolated with N-acetylhomocysteine thiolactone was found by Ellman Assay to have a titer of 0.775 micromoles SH/mL. To 0.5 mL of this thiolated OMPC solution in a ReactiVial was added 0.044 mL of the MPA solution and after ageing for 10 min at room temperature, 0.044 mL of the MPP solution was added. No precipitation of conjugate was noted.

B. Large Scale Experiment:

To 8.5 mL (6.6 micromoles of SH) of the above thiolated OMPC solution was added 0.85 mL of the MPA solution (4.25 micromoles or 65% of MPA) and after ageing for 10 min 0.85 mL of the MPP solution (2.3 micromoles or 35% MPP) was added. The solution was aged at 4°C for 16 hr after which time a precipitate was noticed. The precipitate was resuspended by adjusting the pH to 8.13 with 0.005 mL of 5N NaOH. After ageing for 3 hrs, a small amount of precipitate was removed by a low speed centrifugation. The conjugate was then purified by ultracentrifugation twice at 43K rpm, 4°C for 2 hr. The pellets were resuspended with a Dounce homogenizer in 0.03 M, pH 8 phosphate buffer.

The final conjugate solution was assayed for protein (fd: 0.92 mg/mL) and amino acid analysis (Nle = 60.35 nanomoles/mL, i.e. peptide; 139.6 nanomoles of beta alanine, = total maleimido compounds in conjugate). This corresponds to a 20.4% loading of the peptide by weight onto the protein.

The conjugate was tested in rabbits and found to be efficacious in raising HIV neutralizing antibodies.

EXAMPLE 3

Solid State Synthesis of Disulfide-Bonded cPND4:

A linear PND peptide was prepared on Wang resin using an ABI-431A peptide synthesizer, starting from Fmoc-L-Cys(Acm)-O-Wang resin (0.61 meq/gram). Fmoc chemistry and Fmoc-Amino Acid symmetrical anhydrides (4X excess, prepared in situ) were used as reagents on a 0.25 mmole scale to generate 745 mg of the peptide (SEQ ID: 5:):

```
      Acm                           Mtr
      |                             |
Fmoc-Nle Cys His Ile Gly Pro Gly Arg Ala Phe Cys-O-Wang-resin.
             |                                |
             Trt                              Acm
```

A solution of iodine in 5% methanol/anhydrous DMF (1 ml) was added to the dried, derivatized Wang resin shown above and stirred at room temperature for 4 hours. The resin was filtered, washed with anhydrous DMF (5 x 2 ml), and finally resuspended in DMF (2 ml). Two drops of a 0.1 M solution of sodium thiosulphate in water were added, and stirred for a few seconds. The resin was washed with aqueous 95% DMF (3 x 2 ml), anhydrous DMF (2 ml), methylene chloride (3 x 2 ml), ether (3 x 2 ml) and dried.

The Fmoc and other protecting groups were removed by treatment with 20% piperidine in DMF over 20 minutes, and the resin was washed and dried. The resin was cleaved from the disulfide bonded cyclic peptide by treatment with 95% TFA/4% ethane dithiol/1% thioanisole (1 ml) at room temperature for 6 hours. The solution was filtered, the resin washed with additional 100% TFA (3 x 1 ml), and the combined filtrate was evaporated to dryness. Material that was soluble in ether was removed by extraction (3 x 2 ml) and discarded.

Preparative HPLC using two 2.12 x 25 cm Vydac C18 reverse phase columns in series and a gradient elution of 20 to 24% $CH_3CN$ over 90′ allowed isolation of a sharp peak eluting at 36.66′ under these conditions. Analytical HPLC yielded a single peak upon co-chromatography of a known disulfide bonded cyclic standard with the product obtained from preparative HPLC. FAB-MS gave a $[M+H]^+$ of 1171, which is consistent with the the disulfide bonded cyclic structure cPND4: (SEQ ID: 5:)

```
H-Nle Cys His Ile Gly Pro Gly Arg Ala Phe Cys-COOH
      |                                   |
      CH2 ---------- S ------------- S ---CH2
```

EXAMPLE 4

Protocol for Inoculation of Animals with Maleimidopropionyl-cPND33-OMPC-Maleimidopropionic Acid Coconjugate:

Alum was used as an adjuvant during the inoculation series. The inoculum was prepared by dissolving the coconjugate in physiologic saline at a final conjugate concentration of 300 μg/ml. Preformed alum (aluminum hydroxide gel) was added to the solution to a final level of 500 μg/ml aluminum. The conjugate was allowed to

adsorb onto the alum gel for two hours at room temperature. Following adsorption, the gel with the conjugate was washed twice with physiologic saline and resuspended in saline to a protein concentration of 300 µg/ml.

African green monkeys were individually inoculated with three 300 µg doses or three 100 µg doses of the conjugate either adsorbed onto alum, or formulated with the Ribi adjuvant. Each dose was injected intramuscularly. The doses were delivered one month apart (week 0, 4 and 8). The animals were bled at intervals of two weeks. Serum samples were prepared from each bleed to assay for the development of specific antibodies as described in the subsequent examples.

EXAMPLE 5

Analysis of Sera for Anti-Peptide IgG Antibodies:

Each serum sample is analyzed by enzyme-linked immunoadsorbent assay (ELISA). Polystyrene microtiter plates were coated with 0.5 µg per well of the synthetic peptide (not conjugated to OMPC) in phosphate-buffered physiological saline (PBS) at 4°C. Each well was then washed with PBS containing 0.05% TWEEN-20 (PBS-T). Test serum, diluted serially in PBS-T, was added to the peptide-containing wells and allowed to react with the adsorbed peptide for one hour at 36°C. After washing with PBS-T, alkaline phosphatase-conjugated goat antihuman IgG was added to the test wells and was allowed to react for one hour at 36°C. The wells were then washed extensively in PBS-T. Each well received 0.1% p-nitrophenyl phosphate in 10% diethanolamine, pH 9.8, containing 0.5 mM $MgCl_2 \cdot 6H_20$. The ensuing reaction was allowed to proceed at room temperature for 30 minutes, at which time it was terminated by the addition of 3.0 N NaOH.

The greater the interaction of antibodies in the test serum with the peptide substrate, the greater is the amount of alkaline phosphatase bound onto the well. The phosphatase enzyme mediates the breakdown of p-nitrophenyl phosphate into a molecular substance which absorbs light at a wavelength of 405 nm. Hence, there exists a direct relationship between the absorbance at 405 nm of light at the end of the ELISA reaction and the amount of peptide-bound antibody.

All the monkeys inoculated with the maleimidopropionyl-cPND33-OMPC-maleimidopropionic acid coconjugate developed antibodies specifically capable of binding the peptide.

EXAMPLE 6

Analysis of Sera for Activity which Specifically Neutralizes HIV Infectivity:

Virus-neutralizing activity is determired with an assay described by Robertson et al., J. Virol. Methods 20: 195-202 (1988). The assay measures specific HIV-neutralizing activity in test serum. The assay is based on the observation that MT-4 cells, a human T-lymphoid cell line, are readily susceptible to infection with HIV and, after a period of virus replication, are killed as a result of the infection.

The test serum is treated at 56°C for 60 minutes prior to the assay. This treatment is required to eliminate non-specific inhibitors of HIV replication. Heat treated serum, serially diluted in RPMI-1640 cell culture medium, is mixed with a standard infection dose of HIV. The dose is determined prior to the assay as containing the smallest quantity of virus required to kill all the MT-4 cells in the assay culture after a period of 7-8 days. The serum-virus mixture is allowed to interact for one hour at 37°C. It then is added to $1.0 \times 10^5$ MT-4 cells suspended in RPMI-1640 growth medium supplemented with 10% fetal bovine serum. The cultures are incubated at 37°C in a 5% $CO_2$ atmosphere for 7 days.

At the end of the incubation period, a metabolic dye, DTT, is added to each culture. This dye is yellow in color upon visual inspection. In the presence of live cells, the dye is metabolically processed to a molecular species which yields a blue visual color. Neutralized HIV cannot replicate in the target MT-4 cells and therefore does not kill the cells. Hence, positive neutralization is assessed by the development of blue color following addition of the metabolic dye.

All monkeys immunized with the maleimidopropionyl-cPND33-OMPC-maleimidopropionic acid coconjugate developed specific antibodies capable of neutralizing human immunodeficiency virus as described above.

SEQUENCE LISTING

(1) GENERAL INFORMATION:

(i) APPLICANT: Tolman, Richard L.
Hannah, John
Emini, Emilio A.
Marburg, Stephen
Bondy, Steven S.

(ii) TITLE OF INVENTION: CYCLIC HIV PRINCIPAL NEUTRALIZING DETERMINANT PEPTIDES

(iii) NUMBER OF SEQUENCES: 5

(iv) CORRESPONDENCE ADDRESS:
(A) ADDRESSEE: Merck & Co., Inc.
(B) STREET: P.O. Box 2000
(C) CITY: Rahway
(D) STATE: New Jersey
(E) COUNTRY: USA
(F) ZIP: 07065

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE:
(C) CLASSIFICATION:

(viii) ATTORNEY/AGENT INFORMATION:

(A) NAME: Pfeiffer, Hesna J

(B) REGISTRATION NUMBER: 22640

(C) REFERENCE/DOCKET NUMBER: 18068IB

(ix) TELECOMMUNICATION INFORMATION:

(A) TELEPHONE: 908-594-4251

(B) TELEFAX: 908-594-4720

(2) INFORMATION FOR SEQ ID NO:1:

(i) SEQUENCE CHARACTERISTICS:

(A) LENGTH: 10 amino acids

(B) TYPE: amino acid

(D) TOPOLOGY: both

(ii) MOLECULE TYPE: peptide

(iii) HYPOTHETICAL: NO

(v) FRAGMENT TYPE: internal

(ix) FEATURE:

(A) NAME/KEY: Disulfide-bond

(B) LOCATION: 2..9

(ix) FEATURE:

(A) NAME/KEY: Peptide

(B) LOCATION: 1

(D) OTHER INFORMATION: /label= PEPTIDE

/note= "MAY BE MORE THAN ONE AMINO ACID - SEE DEFINITIONS"

(ix) FEATURE:

(A) NAME/KEY: Peptide

(B) LOCATION: 3

(D) OTHER INFORMATION: /label= PEPTIDE

/note= "MAY BE MORE THAN ONE AMINO ACID - SEE DEFINITIONS"

(ix) FEATURE:

(A) NAME/KEY: Peptide

(B) LOCATION: 8

(D) OTHER INFORMATION: /label= PEPTIDE

/note= "MAY BE MORE THAN ONE AMINO ACID - SEE DEFINITIONS"

(ix) FEATURE:

(A) NAME/KEY: Peptide

(B) LOCATION: 10

(D) OTHER INFORMATION: /label= PEPTIDE

/note= "MAY BE MORE THAN ONE AMINO ACID - SEE DEFINITIONS"

(xi) SEQUENCE DESCRIPTION: SEQ ID NO:1:

```
Xaa Cys Xaa Gly Pro Gly Arg Xaa Cys Xaa
1               5                   10
```

(2) INFORMATION FOR SEQ ID NO:2:

(i) SEQUENCE CHARACTERISTICS:

(A) LENGTH: 4 amino acids

(B) TYPE: amino acid

(D) TOPOLOGY: both

(ii) MOLECULE TYPE: peptide

(v) FRAGMENT TYPE: internal

(xi) SEQUENCE DESCRIPTION: SEQ ID NO:2:

Gly Pro Gly Arg
1

(2) INFORMATION FOR SEQ ID NO:3:

(i) SEQUENCE CHARACTERISTICS:
(A) LENGTH: 14 amino acids
(B) TYPE: amino acid
(D) TOPOLOGY: both

(ii) MOLECULE TYPE: peptide

(ix) FEATURE:
(A) NAME/KEY: Disulfide-bond
(B) LOCATION: 2..13

(ix) FEATURE:
(A) NAME/KEY: Peptide
(B) LOCATION: 3
(D) OTHER INFORMATION: /label= PEPTIDE
/note= "UP TO 8 AMINO ACIDS LONG"

(ix) FEATURE:

(A) NAME/KEY: Peptide

(B) LOCATION: 12

(D) OTHER INFORMATION: /label= PEPTIDE

/note= "UP TO 8 AMINO ACIDS LONG"

(ix) FEATURE:

(A) NAME/KEY: Modified-site

(B) LOCATION: 1

(D) OTHER INFORMATION: /label= nLE

/note= "NORLEUCINE"

(xi) SEQUENCE DESCRIPTION: SEQ ID NO:3:

Leu Cys Xaa Xaa Xaa Gly Pro Gly Arg Xaa Xaa Xaa Cys Xaa

1

5 10

(2) INFORMATION FOR SEQ ID NO:4:

(i) SEQUENCE CHARACTERISTICS:

(A) LENGTH: 26 amino acids

(B) TYPE: amino acid

(D) TOPOLOGY: both

(ii) MOLECULE TYPE: peptide

(ix) FEATURE:

(A) NAME/KEY: Modified-site

(B) LOCATION: 1

(D) OTHER INFORMATION: /label= Nle

/note= "norleucine"

(ix) FEATURE:

(A) NAME/KEY: Disulfide-bond

(B) LOCATION: 2..26

(xi) SEQUENCE DESCRIPTION: SEQ ID NO:4:

```
Leu Cys Tyr Asn Lys Arg Lys Arg Ile His Ile Gly Pro Gly Arg Ala
1               5                   10                  15

Phe Tyr Thr Thr Lys Asn Ile Ile Gly Cys
            20                  25
```

(2) INFORMATION FOR SEQ ID NO:5:

(i) SEQUENCE CHARACTERISTICS:
(A) LENGTH: 11 amino acids
(B) TYPE: amino acid
(D) TOPOLOGY: both

(ii) MOLECULE TYPE: peptide

(ix) FEATURE:
(A) NAME/KEY: Modified-site
(B) LOCATION: 1
(D) OTHER INFORMATION: /label= Nle
/note= "norleucine"

(ix) FEATURE:
(A) NAME/KEY: Disulfide-bond
(B) LOCATION: 2..11

(xi) SEQUENCE DESCRIPTION: SEQ ID NO:5:

```
Leu Cys His Ile Gly Pro Gly Arg Ala Phe Cys
1               5                   10
```

## Claims

1. A cyclic HIV PND peptide having the structure (SEQ ID: 1:):

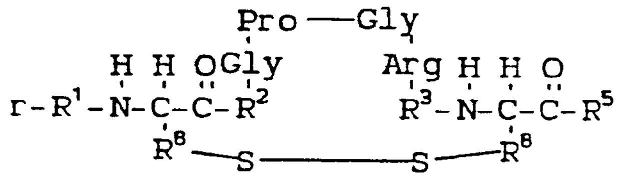

or pharmaceutically acceptable salts thereof, wherein:

r is:

a) hydrogen, or

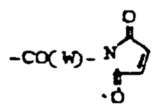 .

wherein W is $-CH_2CH_2-$, $-CH_2CH_2CH_2-$ or $R^6$, where $R^6$ is

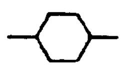 , 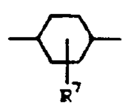 , 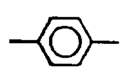 or 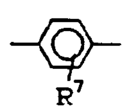

wherein $R^7$ is lower alkyl, lower alkoxy, or halo;

$R^1$ is:

a) a bond, or

b) a peptide of 1 to 5 amino acids, optionallly including a marker amino acid;

$R^2$ is : a peptide of 3 to 10 amino acids

$R^3$ is: a peptide of 3 to 10 amino acids

$R^5$ is:

a) -OH,

b) a peptide of 1 to 5 amino acids, optionally including a marker amino acid, or

c) $-NH_2$;

$R^8$ is a lower alkyl of between one and eight carbons.

2. The peptide of Claim 1 having the structure (SEQ ID: 2:):

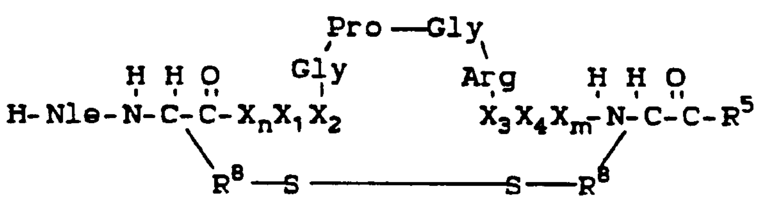

or pharmaceutically acceptable salts thereof,

wherein:

$X_1$ is a constituent of $R^2$ selected from:

a) serine,

b) proline,
c) arginine,
d) histidine,
e) glutamine, or
f) threonine;

$X_2$ is a constituent of $R^2$ selected from:
a) isoleucine,
b) arginine,
c) valine, or
d) methionine;

$X_n$ is is a constituent of $R^2$ and is either an amino acid or a peptide of up to 8 amino acids;
$X_3$ is a constituent of $R^3$ selected from:
a) alanine,
b) arginine, or
c) valine;

$X_4$ is a constituent of $R^3$ and is selected from:
a) phenylalanine,
b) isoleucine,
c) valine, or
d) leucine; and

$X_m$ is a constituent of $R^3$ and is an amino acid or a peptide of up to 8 amino acids.

3. The peptide of Claim 2 having the structure (SEQ ID: 3:):

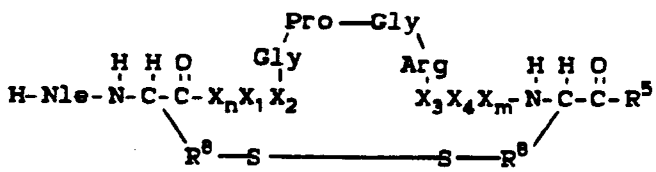

or pharmaceutically acceptable salts thereof, wherein:
$X_1$ is a constituent of $R^2$ selected from:
a) histidine, or
b) glutamine;

$X_2$ is a constituent of $R^2$ and is:
a) isoleucine, or
b) arginine;

$X_n$ is is a constituent of $R^2$ and is either an amino acid or a peptide of up to 8 amino acids;
$X_3$ is a constituent of $R^3$ selected from:
a) alanine,
b) arginine, or
c) valine;

$X_4$ is a constituent of $R^3$ and is selected from:
a) phenylalanine,
b) isoleucine,
c) valine, or
d) leucine;

$X_m$ is a constituent of $R^3$ and is an amino acid or a peptide of up to 8 amino acids.

4. The peptide of Claim 3 having the structure (SEQ ID: 4:):

```
H-Nle Cys Tyr Asn Lys Arg Lys Arg Ile His Ile Gly Pro
      CH2     Gly Arg Ala Phe Tyr Thr Thr Lys Asn Ile
              Ile Gly Cys-OH
      S       S       CH2
```

5. The peptide of Claim 3 having the structure (SEQ ID: 5:):

```
H-Nle Cys His Ile Gly Pro Gly Arg Ala Phe Cys-COOH
      CH2         S                  S        CH2
```

6. A process for making the peptide of Claim 1 which comprises the steps of (a) synthesizing a linear HIV PND peptide containing side-chain protected amino acids and Acm derivatized cysteines on either side of the loop amino acids -$R^2$-Gly-Pro-Gly-Arg-$R^3$-; (b) reacting the Acm derivatized cysteines with iodine; (c) removing all amino acid protecting groups and (d) isolating the disulfide bonded product.

7. The process of Claim 6 for making a peptide having the structure (SEQ ID: 3:):

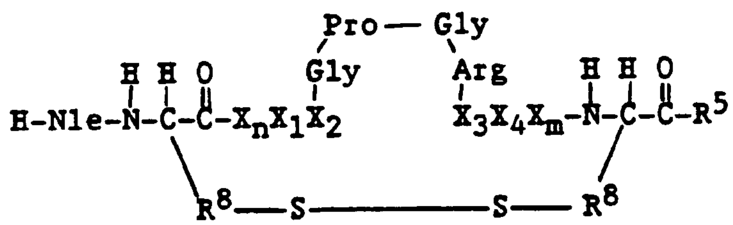

or pharmaceutically acceptable salts thereof, wherein:
$X_1$ is a constituent of $R^2$ selected from:
a) serine,
b) proline,
c) arginine,
d) histidine,
e) glutamine, or
f) threonine;
$X_2$ is a constituent of $R^2$ selected from:
a) isoleucine,
b) arginine,
c) valine, or
d) methionine;
$X_n$ is is a constituent of $R^2$ and is either an amino acid or a peptide of up to 8 amino acids;
$X_3$ is a constituent of $R^3$ selected from:
a) alanine,
b) arginine, or
c) valine;
$X_4$ is a constituent of $R^3$ and is selected from:
a) phenylalanine,
b) isoleucine,
c) valine, or
d) leucine; and
$X_m$ is a constituent of $R^3$ and is an amino acid or a peptide of up to 8 amino acids;
which consists essentially of the steps of (a) preparing a linear, Acm-thiol protected peptide having the structure

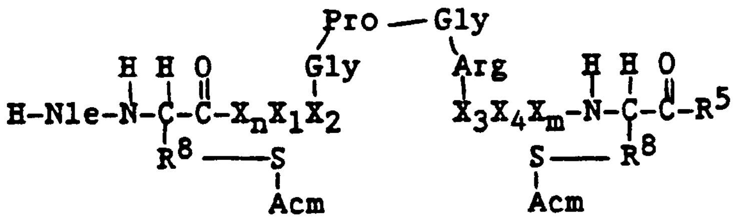

(b) reacting the peptide of step (a) with iodine; (c) removing all amino acid protecting groups; and (d) isolating the disulfide product.

**8.** The process of Claim 7 for making a peptide having the structure:

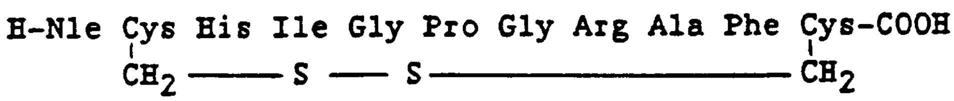

**9.** A process for making the peptide of Claim 1 which comprises the steps of (a) synthesizing a linear HIV PND peptide containing side-chain protected amino acids and having a cysteine on either side of the loop amino acids -$R^2$-GlyProGlyArg-$R^3$; (b) oxidizing the product of step (a) in about 0.1% TFA/20% $CH_3CN$ solution, or by separating oxygen scavangers from the peptide by liquid chromatography in methanol, allowing exposure to the air for about one to fifty hours at between 10 and 40°C.

**10.** The process of Claim 9 for making a peptide having the structure (SEQ ID: 4:):

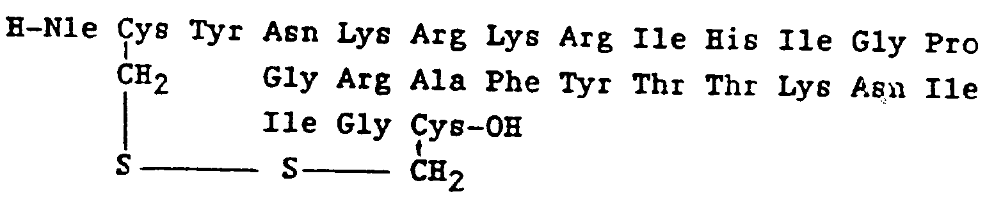

**11.** The use of a compound as claimed in Claim 1 for the manufacture of a medicament for inducing anti-peptide, anti-HIV, or HIV-neutralizing antibodies in a mammal.

**12.** The use of a compound as claimed in Claim 1 for the manufacture of a medicament for the treatment of HIV.